# EUROPÄISCHE PATENTSCHRIFT

(11) **EP 2 361 569 B1**
(45) Veröffentlichungstag und Bekanntmachung des Hinweises auf die Patenterteilung: **31.07.2013**
(21) Anmeldenummer: 11166542.8
(22) Anmeldetag: 27.05.2005
(51) Int. Cl.: A61B 17/68

(54) **Implantat zur Fixierung benachbarter Knochenplatten**
IMPLANT FOR SECURING NEIGHBOURING BONE PLATES
IMPLANT POUR FIXER DES PLAQUES OSSEUSES ADJACENTES

(30) Priorität: 04.08.2004 DE 102004038823
(43) Veröffentlichungstag der Anmeldung: 31.08.2011
(62) Teilanmeldung aus: 05754309.2
(73) Patentinhaber: AESCULAP AG, 78532 Tuttlingen (DE)
(72) Erfinder: Weißhaupt, Dieter, 78194, Immendingen (DE); Steinhilper, Klaus-Dieter, 78532, Tuttlingen (DE); Lerch, Dr. Karl-Dieter, 57439 Attendorn (DE)
(74) Vertreter: Hoeger, Stellrecht & Partner Patentanwälte

(56) Entgegenhaltungen:
- DE-A1- 10 161 724
- DE-C1- 10 128 917
- DE-C1- 19 952 359

## Beschreibung

Die Erfindung betrifft ein Implantat zur Fixierung benachbarter Knochenplatten, insbesondere benachbarter Schädelknochenplatten, wobei die Knochenplatten jeweils eine Innenseite und eine Außenseite aufweisen, umfassend ein inneres Anlageelement zur Anlage an die Innenseite der benachbarten Knochenplatten, ein äußeres Anlageelement zur Anlage an die Außenseiten der benachbarten Knochenplatten und ein linearflexibles Spannelement zur Verbindung des inneren Anlageelements und des äußeren Anlageelements derart, dass diese nicht mehr voneinander entfernbar sind, wobei das äußere Anlageelement mindestens eine Ausnehmung zur mindestens teilweisen Aufnahme eines Verbindungsbereiches zwischen freien Strängen des Spannelements oder zur Durchführung des Verbindungsbereichs in einen Trennspalt zwischen den benachbarten Knochenplatten aufweist, wobei die mindestens eine Ausnehmung so ausgebildet ist, dass der Verbindungsbereich mindestens teilweise unterhalb einer äußeren Oberfläche des äußeren Anlageelements positionierbar ist .

Aus der DE 199 52 359 C1, aus der der Oberbegriff des Hauptanspruchs abgeleitet wird, ist ein solches Implantat bekannt (welches dort als chirurgisches Verbindungselement bezeichnet ist).

Ein weiteres Beispiel eines solchen Implantats ist aus der DE 101 28 917 C1 bekannt.

Aus der US 5,921,986 ist ein Verfahren zur Behandlung eines gebrochenen Knochens bekannt, bei dem ein mit einem Faden verbundener Anker durch den Knochen auf beiden Seiten des Bruchs geführt wird, der Faden gespannt wird, um eine Kraft auf einen Knochen auf der ersten Seite des Bruchs zu übertragen, wobei der Faden sich über den Bruch erstreckt, und bei dem eine Kraft von einem zweiten Anker auf den Knochen auf einer zweiten Seite des Bruchs übertragen wird.

Der Erfindung liegt die Aufgabe zugrunde, ein Implantat der eingangs genannten Art mit verbesserten Eigenschaften bereitzustellen.

Diese Aufgabe wird bei dem eingangs genannten Implantat erfindungsgemäß dadurch gelöst, dass das äußere Anlageelement mindestens zwei beabstandete Durchführungsausnehmungen für das Spannelement aufweist, dass die mindestens eine Ausnehmung für den Verbindungsbereich an einer Durchführungsausnehmung gebildet ist oder eine Durchführungsausnehmung bildet, dass die mindestens eine Ausnehmung an einem äußeren Bereich des äußeren Anlageelements angeordnet ist, und dass das äußere Anlageelement in dem äußeren Bereich verstärkt ist.

Über den Verbindungsbereich werden die freien Stränge des linearflexiblen Spannelements, bei dem es sich insbesondere um einen Faden oder einen Draht handelt, verknüpft. Das entstehende Gebilde, wie beispielsweise ein Knoten, kann zu Irritationen in dem umliegenden Gewebe führen oder es besteht die Gefahr, dass für einen Patienten dieser Verbindungsbereich fühlbar ist oder wird. Erfindungsgemäß ist mindestens eine Ausnehmung vorgesehen, über die der Verbindungsbereich gegenüber einer äußeren Oberfläche des äußeren Anlageelements mindestens teilweise versenkbar ist. Dadurch lässt sich ein Überstehen des Verbindungsbereichs über das äußere Anlageelement verhindern oder zumindest verringern, und damit wiederum lassen sich Gewebeirritationen verhindern oder zumindest verringern.

Die mindestens eine Ausnehmung ist so ausgebildet, dass der Verbindungsbereich mindestens teilweise unterhalb einer äußeren Oberfläche des äußeren Anlageelements positionierbar ist. Dadurch kann der Verbindungsbereich (und insbesondere ein Knoten) mindestens teilweise gegenüber dieser äußeren Oberfläche versenkt werden.

Insbesondere ist der Verbindungsbereich mittels eines Knotens gebildet. Über den Knoten lassen sich die freien Stränge mit einander dauerhaft verbinden und dadurch die Anlageelemente gegeneinander fixieren, so dass diese nicht mehr voneinander entfernbar sind.

Ganz besonders vorteilhaft ist es, wenn der Knoten als Rutschknoten ausgebildet ist. Er lässt sich dann beispielsweise über einen Rutschknotenapplikator, wie er in der DE 101 61 724 A1 beschrieben ist, an eine gewünschte Stelle schieben und zusammenziehen, um eine endgültige Fixierung zu erreichen.

Ganz besonders vorteilhaft ist es, wenn mit oder ohne die Ausnehmung für den Verbindungsbereich der Verbindungsbereich mittels eines vorgefertigten Rutschknotens gebildet ist. Es lässt sich dadurch dem Operateur ein fertig konfektioniertes Implantat bereitstellen, welches auf einfache Weise einsetzbar ist. Der Operateur muss dann nur noch den Rutschknoten an die richtige Stelle positionieren und eine Schlaufe zuziehen. Insbesondere muss er selber keinen Knoten herstellen.

Günstig ist es, wenn die mindestens eine Ausnehmung einen Aufnahmeraum für den Verbindungsbereich aufweist .

Die mindestens eine Ausnehmung ist an einem äußeren Bereich des äußeren Anlageelements angeordnet, und das äußere Anlageelement ist in dem äußeren Bereich verstärkt. Dadurch ist gewährleistet, dass die Ausnehmung nicht die mechanische Stabilität des äußeren Anlageelements beeinträchtigt.

Die freien Stränge sind vorzugsweise diejenigen Bereiche des Spannelements, welche von dem äußeren Anlageelement ausgehend. Durch dauerhafte Verbindung dieser freien Stränge lassen sich die Anlageelemente so gegeneinander fixieren, dass sie nicht auseinander bewegbar sind. Dadurch wiederum lassen sich die Knochenplatten relativ zueinander fixieren.

Es kann vorgesehen sein, dass die mindestens eine Ausnehmung durchgehend ist. Dadurch lässt sich ein Verbindungsbereich und insbesondere ein Knoten durch die Ausnehmung hindurch in den Trennspalt zwischen den Knochenplatten schieben und dort unterbringen, so dass der nicht über eine äußere Oberfläche des äußeren Anlageelements hinausragt.

Es kann vorgesehen sein, dass die mindestens eine Ausnehmung einen Aufnahmeraum für den Verbindungsbereich aufweist. Dieser Aufnahmeraum kann beispielsweise muldenförmig ausgestaltet sein. Über einen solchen Aufnahmeraum lässt sich der Verbindungsbereich versenkt gegenüber einer äußeren Oberfläche des äußeren Anlageelements positionieren.

Beispielsweise ist der mindestens einen Ausnehmung ein Boden zugeordnet. Über diesen Boden lässt sich verhindern, dass der Verbindungsbereich in den Trennspalt geschoben werden kann.

Günstigerweise weist das innere Anlageelement mindestens zwei beabstandete Durchführungsausnehmungen für das Spannelement auf. Über die Durchführungsausnehmungen lässt sich das Spannelement an dem inneren Anlageelement halten und es lässt sich für eine Umlenkung des Spannelements sorgen.

Insbesondere ist das Spannelement an einer Oberfläche des inneren Anlageelements zwischen Durchführungsausnehmungen angelegt. Über den entsprechenden Anlegebereich lässt sich das innere Anlageelement über das Spannelement relativ zu dem äußeren Anlageelement verschieben und es lassen sich die beiden Anlageelemente gegeneinander verspannen.

Das äußere Anlageelement weist mindestens zwei beabstandete Durchführungsausnehmungen für das Spannelement auf. Dadurch lassen sich beispielsweise die freien Stränge des Spannelements durch das äußere Anlageelement durchführen.

Die mindestens eine Ausnehmung für den Verbindungsbereich ist an einer Durchführungsausnehmung gebildet oder bildet eine Durchführungsausnehmung. Dadurch muss eine minimierte Anzahl an Durchbrechungen des äußeren Anlageelements vorgesehen werden, was die mechanische Stabilität erhöht. Weiterhin ist die Herstellung vereinfacht.

Ganz besonders vorteilhaft ist es, wenn das Spannelement so an dem inneren Anlageelement und dem äußeren Anlageelement geführt ist, dass ein Flaschenzugsystem gebildet ist. Dadurch lässt sich zum einen eine sichere Verspannung zwischen den Anlageelementen über das Spannelement erreichen und damit eine sichere Fixierung der benachbarten Knochenplatten. Zum anderen lässt sich eine gezielte Führung des äußeren Anlageelements und des inneren Anlageelements relativ zueinander während eines Spannvorgangs erreichen, wodurch die Arbeit für einen Operateur erleichtert wird. Weiterhin lassen sich relativ große Spannkräfte einbringen, die in großen Klemmkräften der Anlageelemente auf die zwischen ihnen gehaltenen Knochenplatten resultieren. Dadurch ergibt sich ein sicher Halt der Knochenplatten zwischen den Anlageelementen und damit eine sichere Fixierung der Knochenplatten relativ zueinander.

Günstig ist es, wenn in Kombination mit der mindestens einen Ausnehmung für den Verbindungsbereich oder auch ohne eine solche Ausnehmung das innere Anlageelement ein erstes Paar benachbarter Durchführungsausnehmungen für das Spannelement aufweist, zwischen welchen das Spannelement an dem inneren Anlageelement in einem ersten Anlegebereich anliegt, das innere Anlageelement ein zweites Paar benachbarter Durchführungsausnehmungen für das Spannelement aufweist, zwischen welchen das Spannelement am inneren Anlageelement in einem zweiten Anlegebereich anliegt, das äußere Anlageelement ein erstes Paar benachbarter Durchführungsausnehmungen für das Spannelement aufweist, zwischen welchen das Spannelement am äußeren Anlageelement in einem dritten Anlegebereich anliegt und das äußere Anlagebereich ein zweites Paar beabstandeter Durchführungsausnehmungen aufweist, durch welche die freien Stränge des Spannelements durchgeführt sind. Bei einer solchen Anordnung von Durchführungsausnehmungen lässt sich das Spannelement in einer ersten Kehre am inneren Anlageelement führen, dann zum äußeren Anlageelement umlenken, am äußeren Anlageelement in einer weiteren Kehre anlegen und zu dem inneren Anlageelement umlegen und von dort wiederum über eine weitere Kehre anlegen und zum äußeren Anlageelement umlenken. Es lässt sich dadurch ein flaschenzugartiges System ausbilden, welches sich auf vorteilhafter Weise einsetzen lässt. Beispielsweise lassen sich große Klemmkräfte ausüben und es ist für eine sichere Führung des äußeren Anlageelements über das Spannelement gesorgt, so dass der Einsatz für einen Operateur vereinfacht ist.

Insbesondere ist das erste Paar der Durchführungsausnehmungen des äußeren Anlageelements zwischen den Durchführungsausnehmungen des zweiten Paars angeordnet. Dadurch lässt sich eine Kehre des Spannelements zwischen den Durchführungsausnehmungen des ersten Paars ausbilden, um so wiederum ein Flaschenzugsystem ausbilden zu können.

Vorteilhafterweise sind die Durchführungsausnehmungen des inneren Anlageelements auf einer Linie angeordnet. Sie sind dann fluchtend ausgerichtet. Dadurch sind die Spannelementbereiche, welche den Trennspalt durchsetzen, parallel und fluchtend ausgerichtet. Der Einsatz ist dadurch vereinfacht. Beispielsweise können dann auf einfache Weise ein oder mehrere Anlageelemente (welche an dem inneren Anlageelement und/oder äußeren Anlageelement angeordnet sind) verwendet werden, um die gegenüberliegenden Knochenplatten im Bereich des Trennspalts zwischen den Anlageelementen auf Abstand zu halten, so dass das Spannelement sich nicht zwischen den Knochenplatten verklemmen kann. Dadurch wiederum ist sichergestellt, dass beispielsweise bei Ausübung einer Zugkraft auf das Spannelement die Anlageelemente relativ zueinander bewegt werden, um die benachbarten Knochenplatten klemmend zwischen diesen zu halten.

Aus dem gleichen Grund ist es günstig, wenn die Durchführungsausnehmungen des äußeren Anlageelements auf einer Linie angeordnet sind. Günstigerweise sind die Durchführungsausnehmungen des ersten Paars des äußeren Anlageelements und benachbarter Durchführungsausnehmungen des ersten Paars und des zweiten Paars des inneren Anlageelements zueinander angepasst angeordnet. Dadurch lässt sich das Spannelement so führen, dass sich ein Flaschenzugsystem realisieren lässt.

Aus dem gleichen Grund ist es günstig, wenn die Durchführungsausnehmungen des zweiten Paars des äußeren Anlageelements und nicht benachbarte Durchführungsausnehmungen des ersten Paars und des zweiten Paars des inneren Anlageelements zueinander angepasst angeordnet sind.

Günstig ist es, wenn die mindestens eine Ausnehmung für den Verbindungsbereich in einer Durchführungsausnehmung des zweiten Paars des äußeren Anlageelements angeordnet ist oder eine solche Durchführungsausnehmung bildet. Dadurch wird die Ausbildung des Flaschenzugsystems durch die Ausnehmung nicht gestört. Weiterhin lassen sich Durchbrüche in dem äußeren Anlageelement, die grundsätzlich dessen mechanische Stabilität beeinflussen könnten, minimieren.

Es kann vorgesehen sein, dass die mindestens eine Ausnehmung für das Verbindungselement versetzt zu dem ersten Paar der Durchführungsausnehmungen angeordnet ist. Dadurch kann ein Spannelementbereich neben einer Kehre oder Schlaufe des Spannelements, welches an der Oberfläche des äußeren Anlageelements anliegt, versetzt positioniert werden.

Es ist vorgesehen, dass der erste Anlegebereich und der zweite Anlegebereich an einer dem äußeren Anlageelement abgewandten Oberfläche des inneren Anlageelements liegen. Es lässt sich dadurch über das Spannelement das innere Anlageelement gegen das äußere Anlegeelement ziehen.

Es kann auch vorgesehen sein, dass der dritte Anlegebereich an einer dem inneren Anlageelement abgewandten Oberfläche des äußeren Anlageelements liegt. Dadurch lässt sich dann ein Flaschenzugsystem ausbilden. Insbesondere ist das Spannelement durch das äußere Anlageelement verschieblich durchgeführt. Es lässt sich dann eine Zugkraft ausüben, über die der Abstand zwischen dem inneren Anlageelement und dem äußeren Anlageelement verringerbar ist und dabei eine Klemmkraft auf dazwischenliegende Knochenplatten ausübbar ist. Bei einer Verringerung des Abstands wird das Spannelement nachgeführt, um so eine Klemmkraft ausüben zu können.

Insbesondere ist bei Zug am Spannelement das innere Anlageelement gegen das äußere Anlageelement verschieblich.

Günstigerweise ist das Spannelement zur Durchsetzung des Trennspalts zwischen den Knochenplatten vorgesehen. Dieser stellt den entsprechenden Raum bereit, um das Spannelement in dem Bereich zwischen den Anlageelementen aufnehmen zu können.

Ganz besonders vorteilhaft ist es, wenn das innere Anlageelement und/oder das äußere Anlageelement mindestens einen Abstandshalter für die Knochenplatten zum Eintauchen in den Trennspalt aufweist. Dadurch können die Knochenplatten am Trennspalt zwischen den Anlageelementen auf Distanz gehalten werden, und zwar derart, dass das Spannelement frei den Trennspalt durchsetzen kann. Dadurch wiederum wird verhindert, dass das Spannelement zwischen den Knochenplatten eingeklemmt wird. Durch Ausübung beispielsweise einer Zugkraft können dann die Anlageelemente relativ zueinander bewegt werden, um dadurch wiederum Knochenplatten zwischen ihnen einklemmen zu können. Vorzugsweise liegt der mindestens eine Abstandshalter auf einer Linie mit Durchführungsausnehmungen für das Spannelement, um eine freie Führung des Spannelements im Trennspalt zu gewährleisten.

Es kann vorgesehen sein, dass das innere Anlageelement und/oder das äußere Anlageelement derart gewölbt ausgebildet ist, dass zwischen einem Anlagebereich an eine Knochenplatte und einem den Anlagebereich haltenden Bereich ein Zwischenraum gebildet ist. Dadurch ist es beispielsweise möglich, in gewissem Umfange die Höhe des Anlageelements selber über Kraftausübung zu verändern. Beispielsweise wird bei der Fixierung die Höhe verringert. Wenn die Kraft nachgelassen wird, dann erhöht sich die Kraft. Dies kann für eine zusätzlichen Verspannung sorgen, die für eine Verbesserung der Fixierung von Knochenplatten zwischen den Anlageelementen sorgt.

Es ist dann günstig, wenn das innere Anlageelement und/oder das äußere Anlageelement bezogen auf einen Verbindungsbereich elastisch ausgebildet ist. Es lässt sich dadurch eventuell die Verklemmungswirkung verbessern.

Günstig ist es, wenn das innere Anlageelement und/oder das äußere Anlageelement an einem Anlagebereich an einer Knochenplatte eine Grifffläche aufweist. Diese Grifffläche sorgt für einen verbesserten Griff an der Knochenplatte. Die Grifffläche kann beispielsweise über eine Riffelung, Aufrauhung oder Verzahnung gebildet sein.

Insbesondere ist die Grifffläche umlaufend angeordnet, das heißt sie ist um den Umfang des entsprechenden Anlageelements angeordnet. Dadurch ergibt sich eine symmetrische Ausgestaltung des Anlageelements, die dessen Einsatz vereinfacht.

Günstigerweise ist das Spannelement aus einem resorbierbaren Material hergestellt.

Die Erfindung wird in einem Verfahren zur Fixierung benachbarter Knochenplatten verwendet, wobei das innere Anlageelement an die Innenseiten von benachbarten Knochenplatten angelegt ist, und das äußere Anlageelement an die Außenseiten der benachbarten Knochenplatten angelegt ist, bei dem ein linearflexibles Spannelement das innere Anlageelement und das äußere Anlageelement miteinander verbindet, ein Rutschknoten an freien Strängen des Spannelements gebildet ist oder gebildet wird, der Rutschknoten auf das äußere Anlageelement zugeschoben wird, und der Rutschknoten in eine Ausnehmung des äußeren Anlageelements verschoben wird, welche den Rutschknoten so aufnimmt, dass er nicht über das äußere Anlageelement hinausragt.

Die nachfolgende Beschreibung bevorzugter Ausführungsformen dient im Zusammenhang mit der Zeichnung der näheren Erläuterung der Erfindung. Es zeigen:
- Figur 1: eine schematische perspektivische Darstellung eines ersten Ausführungsbeispiels eines erfindungsgemäßen Implantats;
- Figur 2: eine Schnittdarstellung des Implantats gemäß Figur 1 bei fixierten Knochenplatten, wobei ein Knoten in einer Ausnehmung versenkt ist;
- Figur 3: die gleiche Ansicht wie Figur 2, wobei der Knoten in einen Trennspalt zwischen fixierten Knochenplatten gebracht ist;
- Figur 4: eine Schnittansicht eines zweiten Ausführungsbeispiels;
- Figur 5: eine Schnittansicht eines dritten Ausführungsbeispiels;
- Figur 6: eine perspektivische Darstellung einer Variante eines Anlageelements und des Implantats gemäß Figur 1 und
- Figur 7: eine schematische Darstellung eines Ausführungsbeispiels eines Implantats und eines Rutschknotenapplikators.

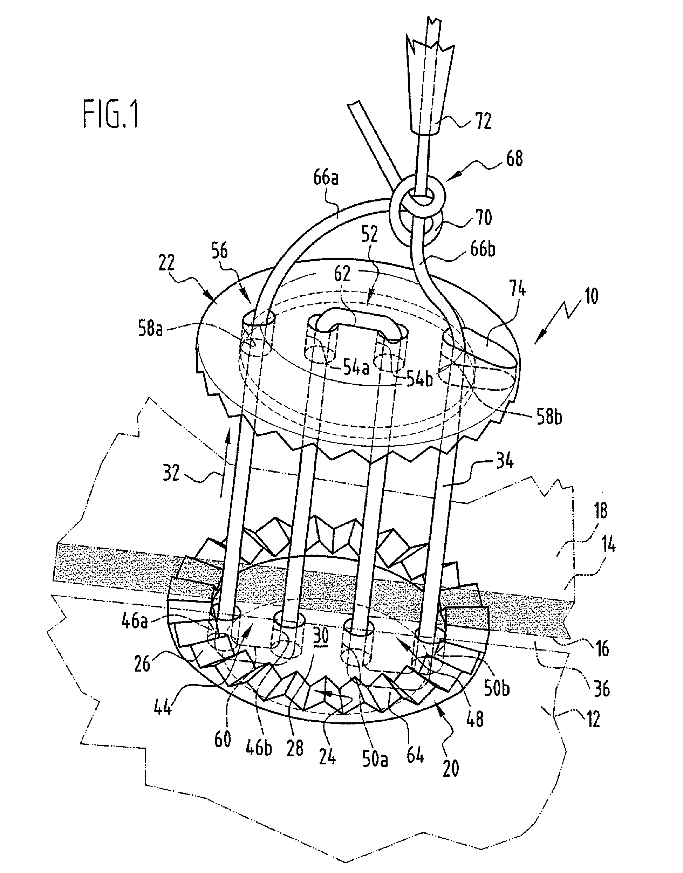

Ein erstes Ausführungsbeispiel eines erfindungsgemäßen Implantats ist in den Figuren 1 bis 3 gezeigt und dort als Ganzes mit 10 bezeichnet. Das Implantat dient zur Fixierung benachbarter Knochenplatten 12, 14. Bei den Knochenplatten 12, 14 handelt es sich insbesondere um Schädelknochenplatten; für Gehirnoperationen wird, um Zugang zum Gehirn zu erhalten, eine Schädelknochenplatte herausgesägt. Nach Beendigung der Gehirnoperation muss diese herausgenommene Schädelknochenplatte (beispielsweise die Knochenplatte 12) wieder an den sie umgebenden Knochenplatten (beispielsweise die Knochenplatte 14 und weitere Knochenplatten) fixiert werden. Dies erfolgt mit einer Mehrzahl von Implantaten 10.

Die Knochenplatten 12, 14 weisen jeweils eine Innenseite 16 und eine gegenüberliegende Außenseite 18 auf. Die Innenseite 16 weist dem Körperinneren zu; bei einer Schädelknochenplatte weist die Innenseite 16 dem Gehirn zu.

Das Implantat 10 umfasst ein inneres Anlageelement 20 zur Anlage an die Innenseite 16 der Knochenplatten 12 und 14. Ferner umfasst das Implantat 10 ein äußeres Anlageelement 22 zur Anlage an die Außenseiten 18 der Knochenplatten 12 und 14.

Bei dem gezeigten Ausführungsbeispiel haben das innere Anlageelement 20 und das äußere Anlageelement 22 einen kreisförmigen Querschnitt. Sie sind bezüglich ihrer Formgestaltung im Wesentlichen gleich ausgebildet.

Das innere Anlageelement 20 sowie das äußere Anlageelement 22 hat einen ringförmigen Anlagebereich, mit dem es an der Innenseite 16 der Knochenplatten 12, 14 anliegt. Dieser Anlagebereich 24 umfasst eine Grifffläche 26, welche "nicht-glatt" ausgebildet ist. Diese Grifffläche 26 dient dazu, den Angriff des inneren Anlageelements 20 an der Innenseite 16 der Knochenplatten 12, 14 bzw. des äußeren Anlageelements 22 an den Außenseiten 18 der Knochenplatten 12, 14 zu verbessern und insbesondere dazu, ein Abrutschen zu verhindern. Beispielsweise ist die Grifffläche 26 mittels einer umlaufenden Verzahnung, Riffelung oder Aufrauhung gebildet.

Das innere Anlageelement 20 (und bei dem gezeigten Ausführungsbeispiel auch das äußeren Anlageelement 22) sind gewölbt ausgebildet, und zwar derart, dass zwischen dem Anlagebereich 24 und einem diesen insbesondere einstückig haltenden Bereich 28 ein Zwischenraum 30 ausgebildet ist.

Es kann vorgesehen sein, dass das innere Anlageelement 20 und/oder das äußere Anlageelement 22 bezogen auf eine Verbindungsrichtung 32 zwischen den beiden Anlageelementen 20 und 22 elastisch ausgebildet sind. Dies kann die Fixierung der Anlageelemente 20 und 22 aneinander und damit die Fixierung von zwischen den Anlageelementen 20 und 22 liegenden Knochenplatten 12 und 14 verbessern.

Zur Verbindung des inneren Anlageelements 20 und des äußeren Anlageelements 22 ist (mindestens) ein linearflexibles Spannelement 34 vorgesehen. Bei dem Spannelement handelt es sich insbesondere um einen chirurgischen Faden oder um einen Draht. Das Spannelement dient dazu, die beiden Anlageelemente 20 und 22 so miteinander zu verbinden, dass diese nicht mehr voneinander entfernbar sind (und damit die Knochenplatten 12 und 14 relativ zueinander zu fixieren), wobei bei Zug am Spannelement 34 das innere Anlageelement 20 gegen das äußeren Anlageelement 22 verschiebbar ist.

Das Spannelement 34 ist insbesondere aus einem im Körper resorbierbaren Material hergestellt.

Das Spannelement 34 durchsetzt zur Fixierung der Knochenplatten 12, 14 einen Trennspalt 36 zwischen den benachbarten Knochenplatten.

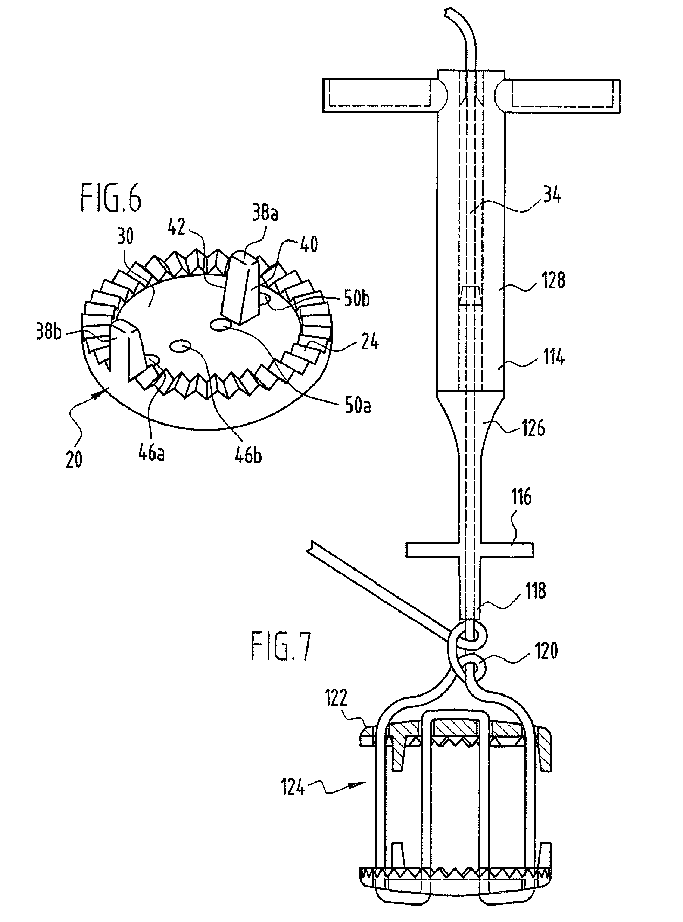

Es kann dabei vorgesehen sein, dass das innere Anlageelement 20 und/oder das äußere Anlageelement 22 mindestens einen Abstandshalter 38a, 38b aufweist (Figur 6), welcher zum Eintauchen in den Trennspalt 36 dient. Die Knochenplatte 12 liegt dann stirnseitig an einer ersten Anlagefläche 40 des bzw. der Abstandshalter 38a, 38b an und die benachbarte Knochenplatte 14 liegt stirnseitig an einer gegenüberliegenden zweiten Anlagefläche 42 des bzw. der Abstandshalter 38a, 38b an. Der bzw. die Abstandshalter 38a, 38b halten die Knochenplatten 12, 14 auf Abstand, damit das Spannelement 34 den Trennspalt 36 so frei durchsetzen kann, dass ein Zug ausübbar ist, um eine Verklemmung des Spannelements 34 im Trennspalt 36 mit den Knochenplatten 12, 14 zu verhindern.

Der oder die Abstandshalter 38a, 38b liegen insbesondere auf einer Linie mit unten noch näher beschriebenen Durchführungsausnehmungen für das Spannelement 34.

Der bzw. die Abstandshalter 38a, 38b sind insbesondere einstückig an dem zugeordneten Anlageelement 20, 22 ausgebildet.

Das innere Anlageelement 20 des Implantats 10 weist ein erstes Paar 44 beabstandeter benachbarter Durchführungsausnehmungen 46a, 46b auf und weist ein zweites Paar 48 beabstandeter benachbarter Durchführungsausnehmungen 50a, 50b auf. Die Durchführungsausnehmungen 46b und 50a sind innenliegende Durchführungsausnehmungen und die Durchführungsausnehmungen 46a, 50b sind außenliegende Durchführungsausnehmungen. Die Durchführungsausnehmung 46b ist benachbart zu der Durchführungsausnehmung 50a. Die Durchführungsausnehmungen 46a und 50b weisen zueinander den größten Abstand zwischen Durchführungsausnehmungen auf. Zwischen den Durchführungsausnehmungen 46a und 50b liegen die Durchführungsausnehmungen 46b und 50a.

Die Durchführungsausnehmungen 46a, 46b, 50a und 50b sind insbesondere auf einer Linie (gemeinsam mit dem oder den Abstandshaltern 38a, 38b) angeordnet, so dass Spannelementbereiche des Spannelements 34, welche zwischen dem inneren Anlageelement 20 und dem äußeren Anlageelement 22 in dem Trennspalt 36 liegen, parallel ausgerichtet zueinander und fluchtend zueinander ausgerichtet positionierbar sind.

Das äußere Anlageelement 22 weist ein erstes Paar 52 von beabstandeten benachbarten Durchführungsausnehmungen 54a, 54b auf. Ferner weist das äußere Anlageelement 22 ein zweites Paar 56 von beabstandeten Durchführungsausnehmungen 58a, 58b auf. Das erste Paar 52 der Durchführungsausnehmungen 54a und 54b liegt dabei zwischen den Durchführungsausnehmungen 58a und 58b. Die Durchführungsausnehmungen 58a, 58b des zweiten Paars 56 sind außenliegenden Durchführungsausnehmungen; die Durchführungsausnehmungen 54a, 54b des ersten Paars 52 sind innenliegende Durchführungsausnehmungen. Die Durchführungsausnehmungen 54a und 58a sind benachbart und die Durchführungsausnehmungen 54b und 58b sind benachbart. Die Durchführungsausnehmungen 58a und 58b des zweiten Paars 56 weisen zueinander den maximalen Abstand zwischen Durchführungsausnehmungen am äußeren Anlageelement 22 auf.

Die Durchführungsausnehmungen 54a, 54b, 58a und 58b des äußeren Anlageelements 22 sind angepasst an die Anordnung der Durchführungsausnehmungen 46a, 46b, 50a, 50b des inneren Anlageelements 20 ausgebildet. Insbesondere weisen benachbarte Durchführungsausnehmungen (58a und 54a; 54a und 54b; 54b und 58b) am äußeren Anlageelement 22 den gleichen Abstand auf wie entsprechende Durchführungsausnehmungen (46a und 46b; 46b und 50a; 50a und 50b) am inneren Anlageelement 20. Weiterhin liegen die Durchführungsausnehmungen 54a, 54b, 58a, 58b des äußeren Anlageelements 22 bevorzugt auf einer Linie.

Durch die angepasste Ausbildung der Durchführungsausnehmungen des inneren Anlageelements 20 und des äußeren Anlageelements 22 lässt sich eine parallele und fluchtende Führung des Spannelements 34 zwischen den Anlageelementen 20 und 22 erreichen.

Das Spannelement 34 ist bei dem in den Figuren 1 bis 3 gezeigten Ausführungsbeispiel einstückig ausgebildet. (Es ist grundsätzlich auch möglich, dass getrennte Spannelemente vorgesehen sind.) Das Spannelement 34 ist durch die Durchführungsausnehmung 54a in dem äußeren Anlageelement 22 geführt und durch die Durchführungsausnehmung 46a im inneren Anlageelement 22. Von der Durchführungsausnehmung 46a ist es zu der Durchführungsausnehmung 46b geführt und durch diese hindurchgeführt. Zwischen den Durchführungsausnehmungen 46a und 46b liegt das Spannelement 34 in einem ersten Anlegebereich 60 an einer äußeren, vom äußeren Anlageelement 22 abgewandten Oberfläche des inneren Anlageelements 20 an. Von der Durchführungsausnehmung 46b ist das Spannelement 34 zu der Durchführungsausnehmung 54a des äußeren Anlageelements 22 geführt und durch diese hindurchgeführt.

Von der Durchführungsausnehmung 54a ist das Spannelement 34 zu der benachbarten Durchführungsausnehmung 54b geführt und durch diese hindurchgeführt. Zwischen den Durchführungsausnehmungen 54a, 54b liegt das Spannelement 34 in einem dritten Anlegebereich 62 an einer äußeren Oberfläche des äußeren Anlageelements 22 an, welche dem inneren Anlageelement 20 abgewandt ist.

Das Spannelement 34 ist durch die Durchführungsausnehmung 54b bis zur Durchführungsausnehmung 50a geführt und durch diese hindurchgeführt. Von der Durchführungsausnehmung 50a ist das Spannelement 34 zur Durchführungsausnehmung 50b geführt und durch diese hindurchgeführt. Zwischen der Durchführungsausnehmung 50a und 50b liegt das Spannelement in einem zweiten Anlegebereich 64 an der äußeren Oberfläche des inneren Anlageelements 20 an.

Von der Durchführungsausnehmung 50b ist das Spannelement 34 zu der Durchführungsausnehmung 58b geführt und durch diese hindurchgeführt.

Ausgehend von den Durchführungsausnehmungen 58a und 58b hat das Spannelement 34 freie Stränge, nämlich einen ersten freien Strang 66a (ausgehend von der Durchführungsausnehmung 58a) und einem freien Strang 66b (ausgehend von der Durchführungsausnehmung 58b).

Wenn über das Implantat 10 die benachbarten Knochenplatten 12 und 14 miteinander fixiert sind, dann liegen die Bereiche des Spannelements 34, welche zwischen den Durchführungsausnehmungen 58a und 46a, 46b und 54a, 54b und 50a sowie 50b und 58b geführt sind, zum größten Teil im Trennspalt 36. Das Spannelement 34 ist über das erste Paar 44 von Durchführungsausnehmungen 46a, 46b am inneren Anlageelement 20 in einer ersten Schlaufe oder Kehre geführt, und das Spannelement 34 von dem inneren Anlageelement 20 ausgehend zu dem äußeren Anlageelement 22 umzulenken.

Durch das erste Paar 52 der Durchführungsausnehmungen 54a, 54b ist das Spannelement 34 in einer zweiten Schlaufe oder Kehre geführt, um das Spannelement 34 vom äußeren Anlageelement 22 zum inneren Anlageelement 20 umzulenken.

Durch das zweite Paar 48 der Durchführungsausnehmungen 50a, 50b am inneren Anlageelement 20 ist das Spannelement 34 in einer dritten Schlaufe oder Kehre geführt, um das Spannelement 34 wiederum vom inneren Anlageelement 20 zum äußeren Anlageelement 22 hin umzulenken.

Die freien Stränge 66a, 66b sind dann über das zweite Paar 56 der Durchführungsausnehmungen 58a, 58b am äußeren Anlageelement 22 aus diesem herausgeführt.

Durch diese Führung des Spannelements 34 an den Anlageelementen 20 und 22 und zwischen den Anlageelementen 20 und 22 ist ein Flaschenzugsystem ausgebildet; wenn beispielsweise an den freien Strängen 66a, 66b gezogen wird (beispielsweise in der Richtung 32), dann wird dadurch das innere Anlageelement 20 in Richtung des äußeren Anlageelements 22 verschoben. Dadurch lässt sich eine Klemmkraft auf zwischen den Anlageelementen 20, 22 liegenden Knochenplatten 12, 14 ausüben, um diese miteinander zu fixieren.

Zur Feststellung dieser Verklemmung derart, dass die Knochenplatten 12, 14 nicht mehr voneinander entfernbar sind, das heißt das innere Anlageelement 20 und das äußere Anlageelement 22 nicht mehr voneinander entfernbar sind, ist eine Verbindung der freien Stränge 66a, 66b des Spannelements 34 vorgesehen. Diese Verbindung erfolgt in einem Verbindungsbereich 68, welcher insbesondere mittels eines Knotens 70 gebildet ist.

Der Knoten 70 ist vorzugsweise als Rutschknoten ausgebildet, welcher beispielsweise über einen Rutschknotenapplikator (Figur 7; in Figur 1 ist eine Beaufschlagungsspitze 72 eines solchen Rutschknotenapplikators angedeutet) auf das äußere Anlageelement 22 zu verschiebbar ist.

Ein Beispiel eines verwendbaren Rutschknotens ist ein Röder Knoten.

Es ist vorzugsweise vorgesehen, dass bei dem Implantat 10 der Knoten 70 und insbesondere Rutschknoten vorgefertigt ist, das heißt das Implantat 10 mit dem inneren Anlageelement 20, dem äußeren Anlageelement 22 und dem wie oben beschrieben geführten Spannelement 34 ausgeführt ist. Ferner sind die freien Stränge 66a, 66b bereits über einen Rutschknoten miteinander verbunden.

Über den Rutschknotenapplikator lässt sich der (Rutsch) Knoten 70 auf das äußere Anlageelement 22 zu verschieben und durch Festziehen beispielsweise über ein freies Ende des Spannelements 34 lassen sich die beiden Anlageelemente 20 und 22 relativ zueinander fixieren.

Wenn das innere Anlageelement 20 und/oder das äußere Anlageelement 22 elastisch ausgebildet sind, dann kann bei der Fixierung eine Kraft ausgeübt werden, welche den Abstand zwischen äußeren Oberflächen des inneren Anlageelements 20 und des äußeren Anlageelements 22 aufgrund elastischer Verformung verringert. Wenn diese Kraft weggenommen ist, dann vergrößert sich dieser Abstand leicht. Es lässt sich dadurch eine Art von Vorspannung ausüben.

Das äußere Anlageelement 22 weist eine Ausnehmung 74 auf, welche zum Aufnehmen bzw. Durchschieben des Verbindungsbereiches 68 des Spannelements 34, das heißt zur Aufnahme bzw. zum Durchschieben des Knotens 70 dient. Die Ausnehmung 74 ist so ausgebildet, dass der Verbindungsbereich 68 mindestens teilweise unterhalb einer äußeren Oberfläche des äußeren Anlageelements 22 positionierbar ist. Dadurch ragt der Verbindungsbereich 68 (insbesondere der Knoten 70) nicht oder nur wenig über diese Oberfläche hinaus. Es lassen sich dann Irritationen des umliegendes Gewebes aufgrund eines auf dem Implantat 10 liegenden Knotens 70 vermeiden, da der Knoten 70 durch die Ausnehmung 74 "versenkbar" ist. Weiterhin ist die Gefahr vermieden, dass der Knoten 70 (als Verbindungsbereich 68) für einen Patienten nach einer bestimmten Zeit fühlbar wird.

Die Ausnehmung 74 ist vorzugsweise an einem äußeren Bereich des äußeren Anlageelements 22 angeordnet, in dem das äußere Anlageelement 22 verstärkt ausgebildet ist. Dadurch ist gewährleistet, dass die Ausnehmung 74 nicht die mechanische Stabilität des äußeren Anlageelements 22 beeinträchtigt.

Vorzugsweise liegt die Ausnehmung 74 an der Durchführungsausnehmung 58b bzw. bildet diese. Die Ausnehmung 74 kann dann direkt als Durchführungsausnehmung genutzt werden. Dadurch verringert sich der Herstellungsaufwand. Weiterhin ist die mechanische Stabilität gewährleistet.

Es ist möglich, dass das äußere Anlageelement 22 auch eine Mehrzahl von solchen Ausnehmungen 74 aufweist. Dies kann beispielsweise notwendig sein, wenn zwei getrennte Spannelemente zur Verbindung der beiden Anlageelemente vorgesehen sind.

Grundsätzlich ist es auch möglich, dass das innere Anlageelement 20 mit einer solchen Ausnehmung 74 oder mehreren Ausnehmungen versehen ist. Es kann vorgesehen sein, dass das innere Anlageelement 20 und das äußere Anlageelement 22 identisch ausgebildet sind. Am inneren Anlageelement 20 hat eine Ausnehmung 74 keine Funktion, wobei sie jedoch auch nicht schädlich für die Fixierung der benachbarten Knochenplatten 12 und 14 ist. Wenn nur ein Typ von Anlageelement hergestellt werden muss, lassen sich die Herstellungskosten verringern und die Herstellung vereinfachen.

Bei dem in den Figuren 1 bis 3 gezeigten Ausführungsbeispiel ist die Ausnehmung 74 durchgehend, das heißt sie geht von einer Oberseite 76 des äußeren Anlageelements 22 zu einer Unterseite 78 des äußeren Anlageelements 22. Es ist dann eine Durchgangsöffnung 80 in dem äußeren Anlageelement 22 gebildet, durch das sich der Knoten 70 durchschieben lässt.

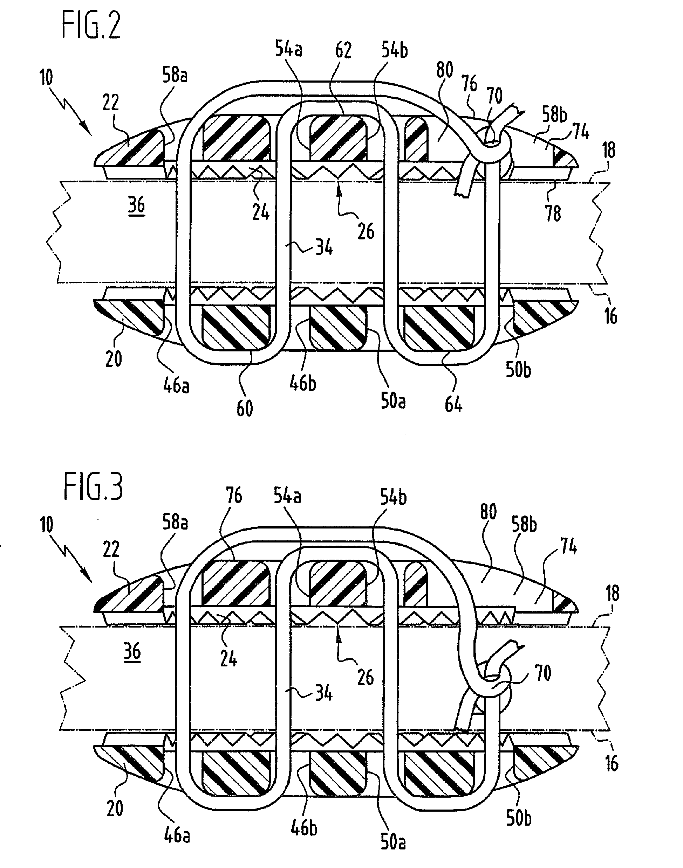

In Figur 2 ist eine Zwischenstellung gezeigt, bei welcher der Knoten 70 in der Durchgangsöffnung 80 liegt. Von dort aus lässt sich der Knoten 70 in den Trennspalt 36 (Figur 3) bringen. Der Knoten 70 ragt dadurch nicht über die Oberseite 76 des Implantats 10 heraus.

Das Implantat 10 funktioniert wie folgt:
Die Anlageelemente 20 und 22 sind über das Spannelement 34 "lose" miteinander verbunden, wobei vorzugsweise ein Rutschknoten 70 an den freien Strängen 66a, 66b vorgefertigt ist. Das innere Anlageelement 20 wird, wenn die benachbarten Knochenplatten 12, 14 miteinander fixiert werden sollen, unter die Knochenplatten 12, 14 gelegt bzw. eine freie Knochenplatte wird auf das positionierte innere Anlageelement 20 aufgelegt.

Das Spannelement 34 verläuft dann in dem Trennspalt 36.

An den freien Strängen 66a, 66b wird gezogen, um den Abstand zwischen dem äußeren Anlageelement 22 und dem inneren Anlageelement 20 zu verringern. Dadurch lässt sich das innere Anlageelement 20 gegen die Innenseiten 16 der Knochenplatten 12, 14 drücken und das äußere Anlageelement 22 gegen die Außenseiten 18 der Knochenplatten 12 und 14.

Zur Fixierung dieser Stellung wird der Rutschknoten 70 beispielsweise über einen Rutschknotenapplikator auf das äußere Anlageelement 22 zu geschoben und dabei in die Ausnehmung 74 verschoben. Von dort aus kann der Knoten 70 in den Trennspalt 36 verschoben werden, so dass er nicht über das äußere Anlageelement 22 hinausragt.

Durch Ziehen an einem freien Ende des Spannelements 34 wird der Knoten 70 zugezogen.

Überstehende Bereiche des Spannelements 34 können dann abgeschnitten werden.

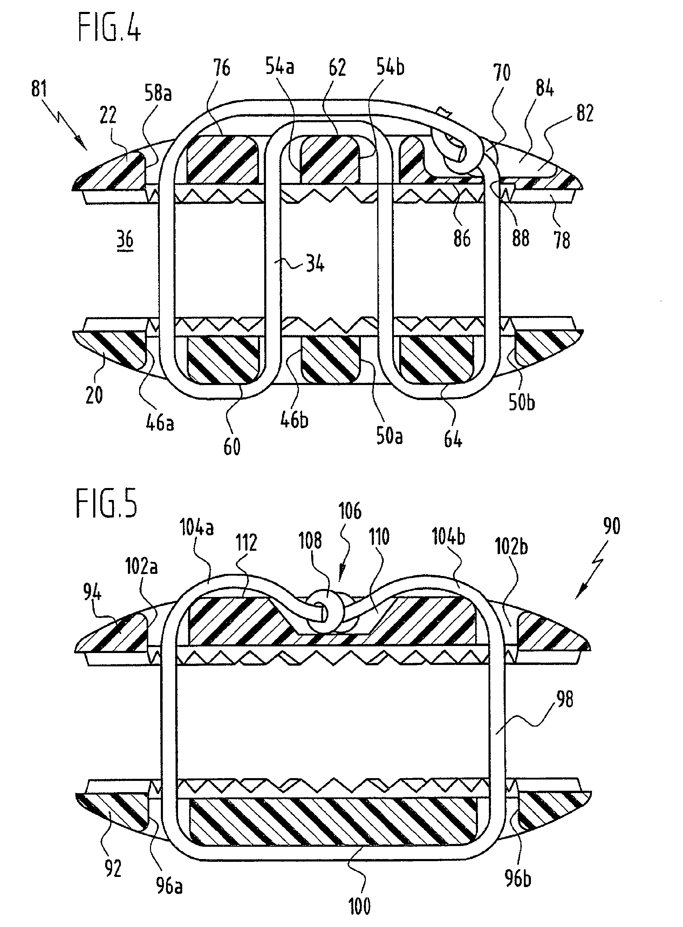

Bei einem zweiten Ausführungsbeispiel 81, welches in einer Schnittdarstellung schematisch in Figur 4 gezeigt ist, ist eine Ausnehmung 82 an dem äußeren Anlageelement 22 vorgesehen, welche nicht durchgehend ist. (Es werden für gleiche Elemente gleiche Bezugszeichen wie für das Ausführungsbeispiel gemäß den Figuren 1 bis 3 verwendet.) Die Ausnehmung 82 weist einen Aufnahmeraum 84 für den Knoten 70 auf. In diesen beispielsweise muldenförmigen Aufnahmeraum 84 ist der Knoten 70 einlegbar, so dass er nicht oder höchstens nur teilweise über die Oberseite 76 des äußeren Anlageelements 22 hinausragt.

Der Ausnehmung 82 und damit dem Aufnahmeraum 84 ist ein Boden 86 zugeordnet, welcher den Aufnahmeraum 84 zu der Unterseite 78 des äußeren Anlageelements 22 verschließt.

In dem Boden 86 ist eine Durchführungsausnehmung 88 gebildet, durch die das Spannelement 34 geführt ist, durch die jedoch der Knoten 70 nicht durchtauchen kann. (Die Durchführungsausnehmung 88 entspricht der Durchführungsausnehmung 58b gemäß dem Ausführungsbeispiel der Figuren 1 bis 3.) Durch die Durchführungsausnehmung 88 ist das Spannelement 34 durch das äußere Anlageelement 22 ausgehend von dem zweiten Anlegebereich 64 am inneren Anlageelement 20 geführt.

Ansonsten funktioniert das Implantat 81 gemäß diesem Ausführungsbeispiel wie oben beschrieben.

Bei einem dritten Ausführungsbeispiel eines Implantats, welches in einer schematischen Schnittdarstellung in Figur 5 gezeigt und dort als Ganzes mit 90 bezeichnet ist, ist ein inneres Anlageelement 92 und ein äußeres Anlageelement 94 vorgesehen. Das innere Anlageelement weist eine erste Durchführungsausnehmung 96a und eine zweite beabstandete benachbarte Durchführungsausnehmung 96b auf. Als Verbindungselement ist wiederum ein linearflexibles Spannelement 98 wie beispielsweise ein Faden oder ein Draht vorgesehen. Dieses Spannelement 98 liegt, wenn die Anlageelemente 92 und 94 miteinander verspannt sind, zwischen den Durchführungsausnehmungen 96a und 96b an einer äußeren Oberfläche des inneren Anlageelements in einem Anlegebereich 100 an.

Das äußere Anlageelement 94 weist angepasst an die Anordnung der Durchführungsausnehmungen 96a, 96b im inneren Anlageelement 92 eine erste Durchführungsausnehmung 102a und eine zweite beabstandete benachbarte Durchführungsausnehmung 102b auf.

Das Spannelement 98 ist von der Durchführungsausnehmung 102a von dem äußeren Anlageelement 94 zu der Durchführungsausnehmung 96a des inneren Anlageelements 92 geführt und durch diese Durchführungsausnehmung 96a durchgeführt. Es ist weiterhin zwischen den Durchführungsausnehmungen 96a und 96b in einer Schlaufe oder Kehre geführt und liegt in dem Anlegebereich 100 an dem inneren Anlageelement 92 an. Es wird an der Durchführungsausnehmung 96b, durch welche es geführt ist, umgelenkt in Richtung des äußeren Anlageelements 94. Dort ist es durch die Durchführungsausnehmung 102b geführt.

Freie Stränge 104a, 104b des Spannelements 98 sind aus den jeweiligen Durchführungsausnehmungen 102a und 102b herausgeführt. Sie sind über einen Verbindungsbereich 106 und insbesondere einen Knoten 108 miteinander verbunden, um die freien Stränge 104a, 104b miteinander zu verbinden und eine Fixierungsstellung der beiden Anlageelemente 92 und 94 zueinander zu sichern.

Zwischen den Durchführungsausnehmungen 102a und 102b ist an dem äußeren Anlageelement 94 eine insbesondere muldenförmige Ausnehmung 110 gebildet. In diese Ausnehmung 110 lässt sich der Knoten 108 einlegen, so dass er nicht oder höchstens teilweise über eine Oberseite 112 des äußeren Anlageelements 94 hinausragt.

Ansonsten funktioniert das Implantat 90 so wie oben anhand der anderen Ausführungsbeispiele beschrieben.

In Figur 7 ist schematisch die Anwendung eines Rutschknotenapplikators 114 angedeutet. Dieser umfasst einen Flansch 116. Ferner umfasst er eine Applikatorspitze 118, mit der auf einen Rutschknoten 120 eine Kraft ausübbar ist, um diesen in Richtung eines äußeren Anlageelements 122 eines Implantats 124 zu verschieben.

Das Implantat 124 ist grundsätzlich so ausgebildet, wie oben beschrieben.

Beispielsweise sitzt die Applikatorspitze 118 an einem Schiebeglied 126 des Rutschknotenapplikators 114. Mit dem Schiebeglied 126 ist ein Griffelement 128 verbunden. Bei einem Ausführungsbeispiel ist die Verbindung derart, dass beim Überschreiten einer bestimmten Verschiebekraft die Verbindung zwischen dem Griffelement 128 und dem Schiebeglied 126 gelöst wird. Ein solcher Rutschknotenapplikator (Vorrichtung zum Zusammenschieben einer Schlaufe eines Fadens, die durch einen längs des Fadens verschiebbaren Rutschknoten des distalen freien Endes des Fadens gebildet wird) ist in der DE 101 61 724 A1 beschrieben. Mit dem Rutschknotenapplikator ist es möglich, das Spannelement 34, welches durch das Griffelement 128 unter das Schiebeglied 126 gefädelt ist, an einem proximalen Ende festzuhalten und über die Applikatorspitze 118 einen Rutschknoten 120 zusammenzuziehen (das heißt aus einer Schlaufe durch Zusammenziehen einen festen Knoten zu bilden).

## Patentansprüche

1. Implantat zur Fixierung benachbarter Knochenplatten (12, 14), insbesondere benachbarter Schädelknochenplatten, wobei die Knochenplatten (12, 14) jeweils eine Innenseite (16) und eine Außenseite (18) aufweisen, umfassend ein inneres Anlageelement (20; 92) zur Anlage an die Innenseiten (16) der benachbarten Knochenplatten (12, 14), ein äußeres Anlageelement (22; 94) zur Anlage an die Außenseiten (18) der benachbarten Knochenplatten (12, 14) und ein linearflexibles Spannelement (34; 98) zur Verbindung des inneren Anlageelements (20; 92) und des äußeren Anlageelements (22; 94) derart, dass diese nicht mehr voneinander entfernbar sind,
wobei das äußere Anlageelement (22; 94) mindestens eine Ausnehmung (74; 82; 110) zur mindestens teilweisen Aufnahme eines Verbindungsbereiches (68; 106) zwischen freien Strängen (66a, 66b; 104a, 104b) des Spannelements (34; 98) oder zur Durchführung des Verbindungsbereichs (68) in einen Trennspalt (36) zwischen den benachbarten Knochenplatten (12, 14) aufweist, wobei die mindestens eine Ausnehmung (74; 82; 110) so ausgebildet ist, dass der Verbindungsbereich (68; 106) mindestens teilweise unterhalb einer äußeren Oberfläche des äußeren Anlageelements (22; 94) positionierbar ist, **dadurch gekennzeichnet, dass** das äußere Anlageelement (22; 94) mindestens zwei beabstandete Durchführungsausnehmungen (54a, 54b, 58a, 58b; 102a, 102b) für das Spannelement (34; 98) aufweist, dass die mindestens eine Ausnehmung (74; 82; 110) für den Verbindungsbereich (68; 106) an einer Durchführungsausnehmung (58b; 88) gebildet ist oder eine Durchführungsausnehmung bildet, dass die mindestens eine Ausnehmung (74; 82) an einem äußeren Bereich des äußeren Anlageelements (22) angeordnet ist, und dass das äußere Anlageelement (22) in dem äußeren Bereich verstärkt ist.

2. Implantat nach Anspruch 1, **dadurch gekennzeichnet, dass** der Verbindungsbereich (68; 106) mittels eines Knotens (70; 108) gebildet ist, und insbesondere dass der Knoten (70; 108) als Rutschknoten ausgebildet ist, und insbesondere dass der Verbindungsbereich (68; 106) mittels eines vorgefertigten Rutschknotens (70; 108) gebildet ist.

3. Implantat nach einem der vorangehenden Ansprüche, **dadurch gekennzeichnet, dass** die mindestens eine Ausnehmung (74) durchgehend ist, oder dass der mindestens einen Ausnehmung (82) ein Boden (86) zugeordnet ist.

4. Implantat nach einem der vorangehenden Ansprüche, **dadurch gekennzeichnet, dass** das Spannelement (34) so an dem inneren Anlageelement (20) und dem äußeren Anlageelement (22) geführt ist, dass ein Flaschenzugsystem gebildet ist.

5. Implantat nach einem der vorangehenden Ansprüche, **dadurch gekennzeichnet, dass** das innere Anlageelement (20) ein erstes Paar (44) beabstandeter Durchführungsausnehmungen (46a, 46b) aufweist, zwischen welchen das Spannelement (34) an dem inneren Anlageelement (20) in einem ersten Anlegebereich (60) anliegt, dass das innere Anlageelement (20) ein zweites Paar (48) benachbarter Durchführungsausnehmungen (50a, 50b) für das Spannelement (34) aufweist, zwischen welchen das Spannelement (34) am inneren Anlageelement (20) in einem zweiten Anlegebereich (64) anliegt, dass das äußere Anlageelement (22) ein erstes Paar (52) benachbarter Durchführungsausnehmungen (54a, 54b) für das Spannelement (34) aufweist, zwischen welchen das Spannelement (34) an dem äußeren Anlageelement (22) in einem dritten Anlegebereich (62) anliegt, und dass das äußere Anlageelement (22) ein zweites Paar (56) beabstandeter Durchführungsausnehmungen (58a, 58b) aufweist, durch welche die freien Stränge (66a, 66b) des Spannelements (34) durchgeführt sind, und insbesondere dass das erste Paar (52) der Durchführungsausnehmungen (54a, 54b) des äußeren Anlageelements (22) zwischen den Durchführungsausnehmungen (58a, 58b) des zweiten Paars (56) angeordnet ist, und insbesondere dass die Durchführungsausnehmungen (46a, 46b, 50a, 50b) des inneren Anlageelements (20) auf einer Linie angeordnet sind, und insbesondere dass die Durchführungsausnehmungen (54a, 54b, 58a, 58b) des äußeren Anlageelements (22) auf einer Linie angeordnet sind, und insbesondere dass die Durchführungsausnehmungen (54a, 54b) des ersten Paars (52) des äußeren Anlageelements (22) und benachbarte Durchführungsausnehmungen (46b, 50a) des ersten Paars (44) und des zweiten Paars (48) des inneren Anlageelements (20) zueinander angepasst angeordnet sind, und insbesondere dass die Durchführungsausnehmungen (58a, 58b) des zweiten Paars (56) des äußeren Anlageelements (22) und nicht benachbarte Durchführungsausnehmungen (46a, 50b) des ersten Paars (44) und des zweiten Paars (48) des inneren Anlageelements (20) zueinander angepasst angeordnet sind, und insbesondere dass die mindestens eine Ausnehmung (74; 82) an einer Durchführungsausnehmung (58b; 88) des zweiten Paars (56) des äußeren Anlageelements (22) angeordnet ist oder eine solche Durchführungsausnehmung bildet, und insbesondere dass die mindestens eine Ausnehmung (74; 82) für den Verbindungsbereich (68) versetzt zu dem ersten Paar (52) der Durchführungsausnehmungen (54a, 54b) angeordnet ist, und insbesondere dass der ersten Anlegebereich (60) und der zweite Anlegebereich (64) an einer dem äußeren Anlageelement (22) abgewandten Oberfläche des inneren Anlageelements (20) liegen, und insbesondere dass der dritte Anlegebereich (62) an einer dem inneren Anlageelement (20) abgewandten Oberfläche des äußeren Anlageelements (22) liegt.

6. Implantat nach einem der vorangehenden Ansprüche, **dadurch gekennzeichnet, dass** das Spannelement (34; 98) durch das äußere Anlageelement (22) verschieblich durchgeführt ist.

7. Implantat nach einem der vorangehenden Ansprüche, **dadurch gekennzeichnet, dass** das Spannelement (34; 98) zur Durchsetzung des Trennspalts (36) zwischen den Knochenplatten (12, 14) vorgesehen ist.

8. Implantat nach einem der vorangehenden Ansprüche, **dadurch gekennzeichnet, dass** das innere Anlageelement (20; 92) und/oder das äußere Anlageelement (22; 94) mindestens einen Abstandshalter (38a, 38b) für die Knochenplatten (12, 14) zum Eintauchen in den Trennspalt (36) aufweist, und insbesondere dass der mindestens eine Abstandshalter (38a, 38b) auf einer Linie mit Durchführungsausnehmungen (46a, 46b, 50a, 50b) liegt.

9. Implantat nach einem der vorangehenden Ansprüche, **dadurch gekennzeichnet, dass** das innere Anlageelement (20; 92) und/oder das äußere Anlageelement (22; 94) derart gewölbt ausgebildet sind, dass zwischen einem Anlagebereich (24) an eine Knochenplatte (12; 14) und einem den Anlagebereich (24) haltenden Bereich (28) ein Zwischenraum (30) gebildet ist, und insbesondere dass das innere Anlageelement (20) und/oder das äußere Anlageelement (22) bezogen auf eine Verbindungsrichtung (32) elastisch ausgebildet ist.

10. Implantat nach einem der vorangehenden Ansprüche, **dadurch gekennzeichnet, dass** das innere Anlageelement (20) und/oder das äußere Anlageelement (22) an einem Anlagebereich (24) an eine Knochenplatte (12; 14) eine Grifffläche (26) aufweist, und insbesondere dass die Grifffläche (26) umlaufend ausgebildet ist.

11. Implantat nach einem der vorangehenden Ansprüche, **dadurch gekennzeichnet, dass** das Spannelement (34; 98) aus einem resorbierbaren Material hergestellt ist.

## Claims

1. Implant for securing neighbouring bone plates (12, 14), especially neighbouring cranial bone plates, the bone plates (12, 14) each having an inner face (16) and an outer face (18), comprising an inner contact element (20; 92) that is adapted to rest on the inner faces (16) of the neighbouring bone plates (12, 14), an outer contact element (22; 94) that is adapted to rest on the outer faces (18) of the neighbouring bone plates (12, 14), and an in a linear manner flexible tensioning element (34; 98) for connecting the inner contact element (20; 92) and the outer contact element (22; 94) in such a manner that they can no longer be separated from one another, the outer contact element (22; 94) comprising at least one recess (74; 82; 110) for at least partially accommodating a connection region (68; 106) between free strands (66a, 66b; 104a, 104b) of the tensioning element (34; 98) or for feeding the connection region (68) through into a separation gap (36) between the neighbouring bone plates (12, 14), and the at least one recess (74; 82; 110) being formed in such a manner that the connection region (68; 106) is positionable at least partially below an outer surface of the outer contact element (22; 94), **characterized in that** the outer contact element (22; 94) comprises at least two spaced feed-through recesses (54a, 54b, 58a, 58b; 102a, 102b) for the tensioning element (34; 98), **in that** the at least one recess (74; 82; 110) for the connection region (68; 106) is formed at a feed-through recess (58b; 88) or forms a feed-through recess, **in that** the at least one recess (74; 82) is arranged at an outer region of the outer contact element (22), and **in that** the outer contact element (22) is strengthened in the outer region.

2. Implant in accordance with claim 1, **characterized in that** the connection region (68; 106) is formed by means of a knot (70; 108), and, in particular, **in that** the knot (70; 108) is in the form of a slip knot, and, in particular, **in that** the connection region (68; 106) is formed by means of a prefabricated slip knot (70; 108).

3. Implant in accordance with any one of the preceding claims, **characterized in that** the at least one recess (74) constitutes a through hole, or **in that** a base (86) is associated with the at least one recess (82).

4. Implant in accordance with any one of the preceding claims, **characterized in that** the tensioning element (34) is guided on the inner contact element (20) and the outer contact element (22) in such a way that a pulley-block system is formed.

5. Implant in accordance with any one of the preceding claims, **characterized in that** the inner contact element (20) comprises a first pair (44) of spaced feed-through recesses (46a, 46b) between which the tensioning element (34) rests on the inner contact element (20) in a first placement region (60), **in that** the inner contact element (20) comprises a second pair (48) of neighbouring feed-through recesses (50a, 50b) for the tensioning element (34) between which the tensioning element (34) rests on the inner contact element (20) in a second placement region (64), **in that** the outer contact element (22) comprises a first pair (52) of neighbouring feed-through recesses (54a, 54b) for the tensioning element (34) between which the tensioning element (34) rests on the outer contact element (22) in a third placement region (62), and **in that** the outer contact element (22) comprises a second pair (56) of spaced feed-through recesses (58a, 58b) through which the free strands (66a, 66b) of the tensioning element (34) are fed, and, in particular, **in that** the first pair (52) of feed-through recesses (54a, 54b) in the outer contact element (22) is arranged between the feed-through recesses (58a, 58b) of the second pair (56), and, in particular, **in that** the feed-through recesses (46a, 46b, 50a, 50b) of the inner contact element (20) are arranged in a line, and, in particular, **in that** the feed-through recesses (54a, 54b, 58a, 58b) of the outer contact element (22) are arranged in a line, and, in particular, **in that** the feed-through recesses (54a, 54b) of the first pair (52) in the outer contact element (22) and neighbouring feed-through recesses (46b, 50a) of the first pair (44) and the second pair (48) in the inner contact element (20) are mutually matched, and, in particular, **in that** the feed-through recesses (58a, 58b) of the second pair (56) in the outer contact element (22) and non-neighbouring feed-through recesses (46a, 50b) of the first pair (44) and the second pair (48) in the inner contact element (20) are mutually matched, and, in particular, **in that** the at least one recess (74; 82) is arranged at a feed-through recess (58b; 88) of the second pair (56) in the outer contact element (22) or forms such a feed-through recess, and, in particular, **in that** the at least one recess (74; 82) for the connection region (68) is arranged such that it is displaced relative to the first pair (52) of feed-through recesses (54a, 54b), and, in particular, **in that** the first placement region (60) and the second placement region (64) are located on a surface of the inner contact element (20) that is remote from the outer contact element (22), and, in particular, **in that** the third placement region (62) is located on a surface of the outer contact element (22) that is remote from the inner contact element (20).

6. Implant in accordance with any one of the preceding claims, **characterized in that** the tensioning element (34; 98) is guided through the outer contact element (22) in displaceable manner.

7. Implant in accordance with any one of the preceding claims, **characterized in that** the tensioning element (34; 98) is provided for passing through the separation gap (36) between the bone plates (12, 14).

8. Implant in accordance with any one of the preceding claims, **characterized in that** the inner contact element (20; 92) and/or the outer contact element (22; 94) comprises at least one spacer (38a, 38b) for the bone plates (12, 14) which is adapted to project into the separation gap (36), and, in particular, **in that** the at least one spacer (38a, 38b) lies along a line with feed-through recesses (46a, 46b, 50a, 50b).

9. Implant in accordance with any one of the preceding claims, **characterized in that** the inner contact element (20; 92) and/or the outer contact element (22; 94) is/are curved in such a manner that an intermediary space (30) is formed between a region of contact (24) with a bone plate (12; 14) and a region (28) incorporating the contact region (24), and, in particular, **in that** the inner contact element (20) and/or the outer contact element (22) is/are resilient taken with reference to a connecting direction (32).

10. Implant in accordance with any one of the preceding claims, **characterized in that** the inner contact element (20) and/or the outer contact element (22) comprises/comprise a gripping area (26) at a region of contact (24) with a bone plate (12; 14), and, in particular, **in that** the gripping area (26) is formed in peripheral manner.

11. Implant in accordance with any one of the preceding claims, **characterized in that** the tensioning element (34; 98) is made of a resorbable material.

## Revendications

1. Implant destiné à la fixation de plaques osseuses (12, 14) voisines, notamment des plaques osseuses ou os plats voisins du crâne, les plaques osseuses (12, 14) présentant chacune un côté intérieur (16) et un côté extérieur (18), l'ensemble comprenant un élément d'appui intérieur (20 ; 92) destiné à venir s'appuyer contre les côtés intérieurs (16) des plaques osseuses (12, 14) voisines, un élément d'appui extérieur (22 ; 94) destiné à venir s'appuyer contre les côtés extérieurs (18) des plaques osseuses (12, 14) voisines, et un élément de serrage (34 ; 98) linéaire, flexible, pour assurer la liaison ou l'assemblage de l'élément d'appui intérieur (20 ; 92) et de l'élément d'appui extérieur (22 ; 94) de manière à ce que ceux-ci ne puissent plus être éloignés l'un de l'autre, l'élément d'appui extérieur (22 ; 94) présentant au moins un évidement (74 ; 82 ; 110) pour y loger au moins partiellement une zone de liaison (68 ; 106) entre des brins libres (66a, 66b ; 104a, 104b) de l'élément de serrage (34 ; 98), ou pour faire passer la zone de liaison (68) dans un interstice de séparation (36) entre les deux plaques osseuses (12, 14) voisines, ledit au moins un évidement (74 ; 82 ; 110) étant configuré de manière à ce que la zone de liaison (68 ; 106) puisse être positionnée au moins partiellement en-dessous d'une surface extérieure de l'élément d'appui extérieur (22 ; 94), **caractérisé en ce que** l'élément d'appui extérieur (22 ; 94) présente au moins deux évidements de passage (54a, 54b, 58a, 58b ; 102a, 102b) mutuellement espacés pour l'élément de serrage (34 ; 98), **en ce que** ledit au moins un évidement (74 ; 82 ; 110) pour la zone de liaison (68 ; 106), est formé au niveau d'un évidement de passage (58b ; 88) ou forme un évidement de passage, **en ce que** ledit au moins un évidement (74 ; 82) est agencé dans une zone extérieure de l'élément d'appui extérieur (22), et **en ce que** 1"élément d'appui extérieur (22) est renforcé dans la zone extérieure.

2. Implant selon la revendication 1, **caractérisé en ce que** la zone de liaison (68 ; 106) est formée au moyen d'un noeud (70 ; 108), et notamment **en ce que** le noeud (70 ; 108) est réalisé en tant que noeud coulant, et notamment **en ce que** la zone de liaison (68 ; 106) est formée au moyen d'un noeud coulant (70 ; 108) préfabriqué.

3. Implant selon l'une des revendications précédentes, **caractérisé en ce que** ledit au moins un évidement (74) est traversant, ou **en ce qu'**au dit au moins un évidement (82) est associé un fond (86).

4. Implant selon l'une des revendications précédentes, **caractérisé en ce que** l'élément de serrage (34) est guidé au niveau de l'élément d'appui intérieur (20) et de l'élément d'appui extérieur (22) de manière à ce que soit formé un système mouflé.

5. Implant selon l'une des revendications précédentes, **caractérisé en ce que** l'élément d'appui intérieur (20) présente une première paire (44) d'évidements de passage (46a, 46b) espacés, entre lesquels l'élément de serrage (34) s'appuie sur l'élément d'appui intérieur (20) dans une première zone d'appui (60), en que l'élément d'appui intérieur (20) présente une deuxième paire (48) d'évidements de passage (50a, 50b) voisins pour l'élément de serrage (34), entre lesquels l'élément de serrage (34) s'appuie sur l'élément d'appui intérieur (20) dans une deuxième zone d'appui (64), **en ce que** l'élément d'appui extérieur (22) présente une première paire (52) d'évidements de passage (54a, 54b) voisins pour l'élément de serrage (34), entre lesquels l'élément de serrage (34) s'appuie sur l'élément d'appui extérieur (22) dans une troisième zone d'appui (62), et **en ce que** l'élément d'appui extérieur (22) présente une deuxième paire (56) d'évidements de passage (58a, 58b) espacés, à travers lesquels sont guidés les brins libres (66a, 66b) de l'élément de serrage (34), et notamment **en ce que** la première paire (52) d'évidements de passage (54a, 54b) de l'élément d'appui extérieur (22) est agencée entre les évidements de passage (58a, 58b) de la deuxième paire (56), et notamment **en ce que** les évidements de passage (46a, 46b, 50a, 50b) de l'élément d'appui intérieur (20) sont agencés sur une ligne, et notamment en ce que les évidements de passage (54a, 54b, 58a, 58b) de l'élément d'appui extérieur (22) sont agencés sur une ligne, et notamment **en ce que** les évidements de passage (54a, 54b) de la première paire (52) de l'élément d'appui extérieur (22), et des évidements de passage voisins (46b, 50a) de la première paire (44) et de la deuxième paire (48) de l'élément d'appui intérieur (20), sont agencés de manière mutuellement adaptée, et notamment **en ce que** les évidements de passage (58a, 58b) de la deuxième paire (56) de l'élément d'appui extérieur (22), et des évidements de passage non voisins (46a, 50b) de la première paire (44) et de la deuxième paire (48) de l'élément d'appui intérieur (20), sont agencés de manière mutuellement adaptée, et notamment **en ce que** ledit au moins un évidement (74 ; 82) est agencé au niveau d'un évidement de passage (58b ; 88) de la deuxième paire (56) de l'élément d'appui extérieur (22), ou forme un tel évidement de passage, et notamment **en ce que** ledit au moins un évidement (74 ; 82) pour la zone de liaison (68), est agencé de manière décalée par rapport à la première paire (52) d'évidements de passage (54a, 54b), et notamment **en ce que** la première zone d'appui (60) et la deuxième zone d'appui (64) se situent sur une surface de l'élément d'appui intérieur (20), qui n'est pas dirigée vers l'élément d'appui extérieur (22), et notamment **en ce que** la troisième zone d'appui (62) se situe sur une surface de l'élément d'appui extérieur (22), qui n'est pas dirigée vers l'élément d'appui intérieur (20).

6. Implant selon l'une des revendications précédentes, **caractérisé en ce que** l'élément de serrage (34 ; 98) est guidé de manière coulissante à travers l'élément d'appui extérieur (22).

7. Implant selon l'une des revendications précédentes, **caractérisé en ce que** l'élément de serrage (34 ; 98) est prévu pour traverser l'interstice de séparation (36) entre les plaques osseuses (12, 14).

8. Implant selon l'une des revendications précédentes, **caractérisé en ce que** l'élément d'appui intérieur (20 ; 92) et/ou l'élément d'appui extérieur (22 ; 94) comporte au moins une entretoise d'espacement (38a, 38b) pour les plaques osseuses (12, 14), qui plonge dans l'interstice de séparation (36), et notamment **en ce que** ladite au moins une entretoise d'espacement (38a, 38b) se situe sur une ligne avec des évidements de passage (46a, 46b, 50a, 50b).

9. Implant selon l'une des revendications précédentes, **caractérisé en ce que** l'élément d'appui intérieur (20 ; 92) et/ou l'élément d'appui extérieur (22 ; 94) sont d'une configuration bombée telle, qu'entre une zone d'appui (24) contre une plaque osseuse (12 ; 14) et une zone (28) supportant la zone d'appui (24), soit formé un espace intermédiaire (30), et notamment **en ce que** l'élément d'appui intérieur (20) et/ou l'élément d'appui extérieur (22) est d'une configuration élastique relativement à une direction de liaison (32).

10. Implant selon l'une des revendications précédentes, **caractérisé en ce que** l'élément d'appui intérieur (20) et/ou l'élément d'appui extérieur (22) présente, sur une zone d'appui (24) contre une plaque osseuse (12 ; 14), une surface agrippante de prise (26), et notamment **en ce que** la surface agrippante de prise (26) est réalisée de manière périphérique.

11. Implant selon l'une des revendications précédentes, **caractérisé en ce que** l'élément de serrage (34 ; 98) est fabriqué en un matériau résorbable.
